# FASCICULE DE BREVET EUROPEEN

(11) **EP 0 940 152 B1**
(45) Date de publication et mention de la délivrance du brevet: **07.07.2004**
(21) Numéro de dépôt: 99810191.9
(22) Date de dépôt: 04.03.1999
(51) Int. Cl.: A61M 3/02, E03C 1/06

(54) **Pomme de douche**
Brausekopf
Shower head

(30) Priorité: 06.03.1998 CH 54798
(43) Date de publication de la demande: 08.09.1999
(73) Titulaire: FRANCINA Sàrl, 1870 Monthey (CH)
(72) Inventeur: Moren, Francine, 1820 Veytaux (CH)
(74) Mandataire: BOVARD AG - Patentanwälte

(56) Documents cités:
- DE-C- 637 278
- FR-A- 2 596 648
- US-A- 3 921 635

## Description

La présente invention concerne une pomme de douche selon le préambule de la revendication 1. La présente invention concerne plus particulièrement une pomme de douche apte à être connectée à l'extrémité libre d'une tuyauterie de douche et comprenant une canule dont la forme permet de l'introduire dans le rectum pour le rinçage intestinal. La présente invention concerne également un assemblage comportant une telle pomme de douche et un support de pomme de douche, ainsi qu'une canule de remplacement pour une telle pomme de douche.

Les déchets stagnant dans l'intestin, en particulier dans la portion rectale de l'intestin, sont à l'origine des pires affections et des maladies les plus diverses. Le livre du Dr. C. Schaller et al., « Hygiène intestinale : clé de la grande forme », paru aux éditions Vivez Soleil, ISBN 2-88058-093-5, explique l'importance primordiale et méconnue d'une hygiène intestinale rigoureuse.

On connaît donc des techniques de lavement intestinal, généralement pratiquées en milieu hospitalier, qui consistent en l'injection d'un volume d'eau important, par exemple plus d'un litre, par une canule introduite profondément dans l'orifice rectal. La longueur de la canule est généralement importante, le plus souvent largement supérieure à 10 centimètres. Cette opération fortement intrusive provoque souvent des gonflements et des nausées chez le sujet, et son usage est donc réservé à des applications thérapeutiques particulières, par exemple à un rinçage pré-opératoire, sous contrôle médical. Les appareils utilisés pour ces lavements sont donc totalement inadaptés à un usage domestique régulier lors de la toilette.

Le document de brevet FR2316920 décrit une canule pour douche vaginale et rectale pouvant être branchée directement sur la tuyauterie d'une douche. Ce dispositif relativement peu coûteux et simple à utiliser permet donc d'effectuer un lavage intestinal à domicile, à l'occasion de la douche, en remplaçant la pomme de douche conventionnelle par la canule décrite.

Le dispositif décrit se fixe directement sur l'extrémité libre du tuyau de douche. Dans le cas d'un tuyau en matériau synthétique, il est donc nécessaire de démonter complètement la pomme de douche ordinaire, d'enfiler la canule de l'invention sur l'extrémité du tuyau, et d'assurer le raccordement en vissant une bride de fixation par-dessus la canule. Cette opération relativement fastidieuse doit être répétée à chaque utilisation, car la canule décrite, qui comporte des orifices relativement peu nombreux, ne permet pas de doucher le reste du corps. L'extrémité du tuyau flexible risque d'être endommagé rapidement en cas de remplacement quotidien de la pomme de douche par la canule décrite. En outre, aucun moyen n'est prévu pour assurer le rinçage de la canule après utilisation. Comme la canule comporte plusieurs pièces indépendantes vissées entre elles, une propreté méticuleuse de l'ensemble du dispositif ne peut être obtenue qu'au moyen d'un démontage et d'un nettoyage fastidieux de la canule après utilisation.

DE637278 décrit une pomme de douche destinée au traitement des hémorroïdes et comprenant une canule rectale rigide relativement longue. Ce dispositif convient bien à un usage médical. Un utilisateur moins expérimenté risque cependant de se blesser lors de l'introduction de cette canule.

Un but de l'invention est donc de proposer une pomme de douche pour lavage intestinal améliorée par rapport aux dispositifs de l'art antérieur.

Selon l'invention, ce but est atteint au moyen des éléments de la partie caractérisante de la revendication 1, des variantes préférentielles de réalisation étant par ailleurs indiquées dans les revendications dépendantes. Une canule de remplacement pour pomme de douche selon l'invention est décrite dans la revendication 6.

Un autre but est de proposer un assemblage formé d'une pomme de douche et d'un support de pomme de douche amélioré par rapport aux dispositifs de l'art antérieur.

Selon l'invention, ce but est atteint au moyen des éléments de la partie caractérisante de la revendication 8, des variantes préférentielles de réalisation étant par ailleurs indiquées dans les revendications dépendantes 9 et 10.

L'invention sera mieux comprise à l'aide de la description donnée à titre d'exemple non limitatif et illustrée par les figures annexées qui montrent :

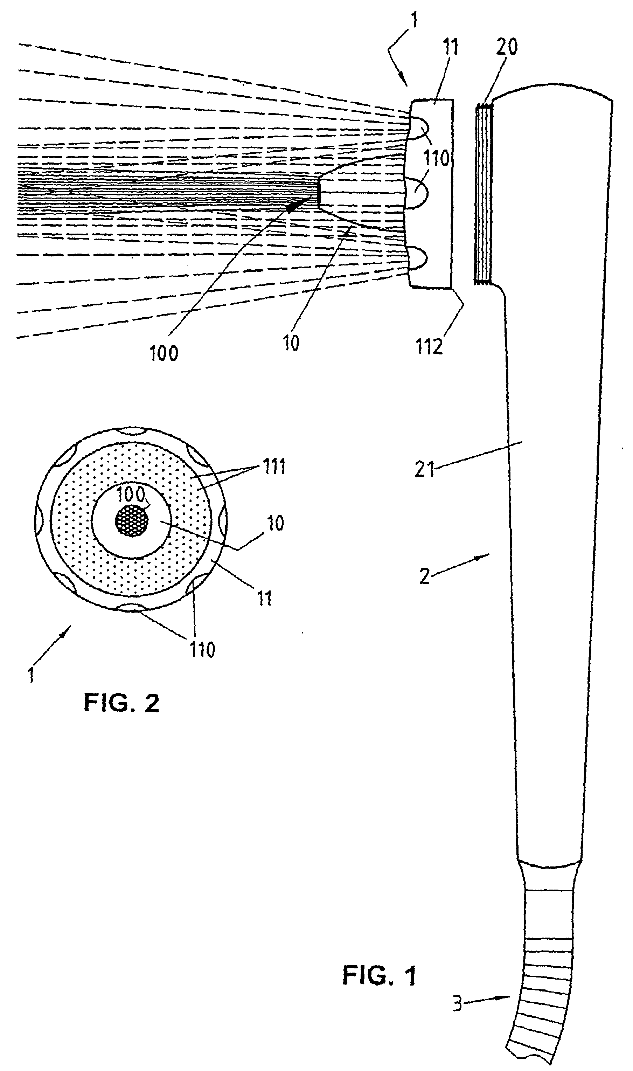

La figure 1 une vue latérale d'une pomme de douche et d'un support de pomme de douche selon l'invention.
La figure 2 une vue de devant d'une pomme de douche selon l'invention.

L'assemblage selon l'invention comporte pour l'essentiel un support de pomme de douche 2 apte à être raccordé par des moyens appropriés à l'extrémité libre d'un tuyau flexible de douche 3, ainsi qu'une pomme de douche 1. Le support de pomme de douche 2 comporte une portion de saisie 21 dans le prolongement du tuyau 3, suffisamment long pour pouvoir le saisir manuellement afin de contrôler l'orientation du jet d'eau, ainsi qu'une portion de raccordement 20 permettant de fixer la pomme de douche 1 de manière amovible. La portion de raccordement 20 comporte de préférence un filetage ou des moyens d'encliquetage, tout autre moyen approprié pouvant être adopté par l'homme du métier. Le support de pomme de douche 2 est de préférence en matériau synthétique ou éventuellement en métal inoxydable, par exemple en aluminium. De préférence, le support de pomme de douche est de type conventionnel standardisé, et permet donc également le raccordement amovible de pommes de douche conventionnelles (non illustrées).

La pomme de douche amovible 1, qui peut être commercialisée en combinaison avec un support 2 adapté ou indépendamment, peut être fixée perpendiculairement au corps du support de douche 2 grâce à des moyens de fixation amovibles 112 aptes à coopérer avec les moyens 20. Les moyens de fixation 112 sont de préférence de type standardisé de manière à pouvoir utiliser la pomme de douche 1 de l'invention avec un grand nombre de supports de pomme de douche. Des moyens de saisie 110 sur le corps 11 de la pomme de douche 1 permettent de faciliter son assemblage par vissage ou encliquetage sur le support 2. Selon l'invention, la pomme de douche 1 comporte une canule centrale proéminente 10 dont la forme par exemple tronconique permet de l'introduire par l'ouverture rectale de l'utilisateur. La canule 10 est munie d'une seule ouverture 100 par laquelle s'écoule l'eau provenant du flexible 3. Dans cet exemple, l'ouverture centrale est de forme circulaire avec un diamètre assez important, par exemple entre 0,5 et 1,0 centimètre. La canule a de préférence une longueur de quelques centimètres, par exemple comprise entre 3 et 4 centimètres, afin d'éviter tout risque de blessure ou de désagrément causé par une insertion trop profonde. La canule peut aussi être réalisée dans un matériau synthétique ou caoutchouté souple plutôt que dans un matériau rigide.

Selon l'invention, la pomme de douche 1 comporte en outre une pluralité d'ouvertures supplémentaires 111 disposées tout autour de la canule 10. Les ouvertures 111 sont reliées à la tuyauterie 3 de manière à ce qu'une partie de l'eau de douche s'écoule par l'ouverture 100 et le reste par les ouvertures périphériques 111, quelle que soit la pression d'eau dans le flexible. Les ouvertures 111 sont disposées de façon à rincer abondamment la portion externe de la canule 10 afin de la maintenir propre. Le débit et la pression de l'eau s'écoulant par les ouvertures 111 sont suffisants pour permettre un douchage corporel normal, un seul jet relativement faible s'écoulant par l'ouverture centrale 100. De cette façon, il est possible de doucher tout le corps et de rincer l'intestin avec la même pomme de douche 1 qui peut restée raccordée en permanence sur le support 2.

La pomme de douche 1 peut être réalisée en une seule pièce. Dans une variante préférentielle toutefois, la canule 10 peut être remplacée périodiquement en la désassemblant du corps de la pomme de douche 11. Le corps 11 peut par exemple comporter un trou central dans lequel la canule relativement molle peut être introduite par l'arrière en la déformant, la canule étant maintenue dans le trou lorsqu'elle reprend sa forme initiale. D'autres moyens de fixation amovible de la canule 10 sur le corps 11 peuvent être facilement imaginés tout en restant dans le cadre de cette invention. Dans ce cas, des canules de remplacement amovibles 10 peuvent aussi être commercialisées pour elles-mêmes, par exemple en emballage multiple.

Pour procéder au lavage rectal, l'utilisateur vérifie tout d'abord la température de l'eau sur sa main, puis réduit le débit à un mince jet sous pression s'écoulant par l'ouverture centrale 100 et par les ouvertures 111. La canule 10 peut ensuite être introduite dans le rectum, sans risque de blessure ou de désagrément en raison de sa longueur réduite et du matériau souple utilisé. Un rinçage très bref est généralement suffisant, la pression de l'eau tiède permettant un nettoyage efficace du côlon. Simultanément, l'eau éjectée par les ouvertures 111 permet de nettoyer l'ensemble de la région anale et génitale externe.

Sitôt extraite, la canule 10 est abondamment rincée par l'eau s'écoulant des ouvertures 111, en sorte qu'une hygiène rigoureuse du dispositif peut être garantie sans intervention de l'utilisateur. L'utilisateur peut ensuite poursuivre sa douche corporelle externe en rétablissant une pression d'eau supérieure, sans remplacer la pomme de douche 1.

L'homme du métier pourra dans le cadre de cette invention réaliser différentes variantes de pommes de douche adaptées à un usage vétérinaire par exemple.

## Revendications

1. Pomme de douche (1) apte à être connectée à l'extrémité libre (20) d'une tuyauterie de douche (2, 3) et comprenant,
. une canule (10) dont la forme permet de l'introduire par l'ouverture rectale, la canule (10) étant munie d'une seule ouverture centrale (100) reliée à ladite tuyauterie de façon à permettre l'injection d'eau dans le rectum,
. une pluralité d'ouvertures supplémentaires, dites ouvertures périphériques (111) disposées tout autour de ladite canule (10) et reliées à ladite tuyauterie de façon à expulser une portion de l'eau à l'extérieur de ladite canule (10),
- les ouvertures périphériques (111) sont reliées à la tuyauterie (3) de manière à ce qu'une partie de l'eau de douche s'écoule par l'ouverture centrale (100) et le reste par lesdites ouvertures périphériques (111), indépendamment de la pression d'eau dans le flexible,
- un seul jet s'écoule par l'ouverture centrale (100), lorsque le débit et la pression de l'eau qui s'écoulent par les ouvertures périphériques (111) sont suffisants pour permettre un douchage corporel.

2. Pomme de douche selon la revendication 1 **caractérisée en ce que** les ouvertures périphériques (111) sont disposées de façon à rincer abondamment la portion externe de la canule (10) afin de la maintenir propre.

3. Pomme de douche selon la revendication 1 ou 2 **caractérisée en ce que** le diamètre de l'ouverture centrale (100) est compris entre 0,5 centimètre et 1,0 centimètre.

4. Pomme de douche (1) selon l'une des revendications précédentes, **caractérisée en ce que** la longueur de ladite canule (10) est comprise entre 3 et 4 centimètres.

5. Pomme de douche (1) selon l'une des revendications précédentes, **caractérisée en ce qu'**elle comporte des moyens de fixation amovible (112) sur un support (2) de pomme de douche.

6. Pomme de douche selon la revendication précédente, **caractérisée par** des moyens de prise manuelle (110) facilitant la fixation sur ledit support de pomme de douche (2).

7. Pomme de douche selon l'une des revendications précédentes, **caractérisée en ce que** ladite canule (10) peut être désolidarisée du corps de la pomme de douche (11) pour la remplacer.

8. Pomme de douche (1) selon l'une des revendications précédentes, **caractérisée en ce que** ladite canule (10) est constituée d'un matériau synthétique souple.

## Patentansprüche

1. Brausekopf (1), der mit dem freien Ende (20) eines Duschschlauches (2, 3) verbindbar ist, umfassend
- eine Kanüle (10), deren Form es erlaubt, diese durch die rektale Öffnung einzuführen, und welche Kanüle (10) mit einer einzigen zentralen Öffnung (100) ausgestattet ist, verbunden mit dem genannten Schlauch, welche eine Einspritzung von Wasser in den Rektum erlaubt,
- eine Mehrzahl von zusätzlichen Öffnungen (111), die peripherisch um die genannte Kanüle (10) angeordnet sind und mit dem genannten Schlauch verbunden sind, wodurch ein Teil des Wassers ausserhalb der genannten Kanüle (10) ausströmt,
- die peripheren Öffnungen (111) mit dem Schlauch (3) so verbunden sind, dass ein Teil des Duschwassers durch die zentrale Öffnung (100) ausströmt und der Rest durch die genannten peripheren Öffnungen (111) ausströmt, unabhängig vom Druck des Wassers im Schlauch,
- und ein einziger Strom strömt durch die zentrale Öffnung (100), während der Ausstoss und der Druck des Wassers, das durch die peripheren Öffnungen (111) austritt, genügend sind für ein körperliches Duschen.

2. Brausekopf nach Anspruch 1, **dadurch gekennzeichnet, dass** die peripheren Öffnungen (111) so angeordnet sind, dass sie den äusseren Bereich der Kanüle (10) reichlich abspülen, um diesen sauber zu halten.

3. Brausekopf nach Anspruch 1 oder 2, **dadurch gekennzeichnet, dass** der Durchmesser der zentralen Öffnung (100) zwischen 0,5 Zentimeter und 1,0 Zentimeter liegt.

4. Brausekopf nach einem der vorhergehenden Ansprüche, **dadurch gekennzeichnet, dass** die Länge der genannten Kanüle (10) zwischen 3 und 4 Zentimeter liegt.

5. Brausekopf nach einem der vorhergehenden Ansprüche, **dadurch gekennzeichnet, dass** dieser abnehmbare Befestigungsmittel (112) auf einem Halter (2) des Brausekopfs aufweist.

6. Brausekopf nach dem vorhergehenden Anspruch, **gekennzeichnet durch** manuelle Griffmittel (110), welche die Befestigung auf dem genannten Halter (2) des Brausekopfes erleichtern.

7. Brausekopf nach einem der vorhergehenden Ansprüche, **dadurch gekennzeichnet, dass** die genannte Kanüle (10) vom Körper des Brausekopfes (11) getrennt werden kann, um sie zu ersetzen.

8. Brausekopf nach einem der vorhergehenden Ansprüche, **dadurch gekennzeichnet, dass** die genannte Kanüle (10) aus einem weichen synthetischen Material gebildet ist.

## Claims

1. Shower head (1) able to be connected to the free end (20) of a shower pipework (2, 3) and comprising
• a hollow needle (10) the form of which permits introduction thereof through the rectal opening, the hollow needle (10) being equipped with a sole central aperture (100) connected to said pipework in a manner so as to permit the injection of water into the rectum,
• a plurality of supplementary apertures, referred to as peripheral apertures (111), all disposed about said hollow needle (10) and connected to said pipework in a manner so as to expel a portion of the water outside of said hollow needle (10),
- the peripheral apertures (111) are connected to the pipework (3) in a manner such that part of the water of the shower flows out through the central aperture (100) and the rest through said peripheral apertures (111), independently of the water pressure in the flexible part,
- a sole jet flows out through the central aperture (100) when the discharge and the pressure of the water coming out through the peripheral apertures (111) are sufficient to permit a corporal showering.

2. Shower head according to claim 1, **characterised in that** the peripheral apertures (111) are disposed in a manner so as to rinse abundantly the outer portion of the hollow needles (10) in order to keep it clean.

3. Shower head according to claim 1 or 2, **characterised in that** the diameter of the central aperture (100) ranges between 0.5 centimetre and 1.0 centimetre.

4. Shower head (1) according to one of the preceding claims, **characterised in that** the length of said hollow needle (10) ranges between 3 and 4 centimetres.

5. Shower head (1) according to one of the preceding claims, **characterised in that** it includes removable fixing means (112) on a shower head support (2).

6. Shower head according to the preceding claim, **characterised by** manual grasping means (110) facilitating the fixing on said shower head support (2).

7. Shower head according to one of the preceding claims, **characterised in that** said hollow needle (10) can be disunited from the shower head body (11) for replacing it.

8. Shower head (1) according to one of the preceding claims, **characterised in that** said hollow needle (10) is made up of a flexible synthetic material.
